# EUROPEAN PATENT SPECIFICATION

(11) **EP 2 958 604 B1**
(45) Date of publication and mention of the grant of the patent: **21.08.2019**
(21) Application number: 14706549.4
(22) Date of filing: 21.02.2014
(51) Int. Cl.: A61L 27/28, A61L 31/08

(54) **MEDICAL DEVICE WITH A BIOCOMPATIBLE COATING**
MEDIZINISCHE VORRICHTUNG MIT BIOKOMPATIBLER BESCHICHTUNG
DISPOSITIF MÉDICAL AVEC UN REVÊTEMENT BIOCOMPATIBLE

(30) Priority: 22.02.2013 EP 13156274
(43) Date of publication of application: 30.12.2015
(73) Proprietor: Cardiatis S.A., 5032 Isnes (BE); Lylyk, Pedro, C1428ARJ Buenos Aires (AR)
(72) Inventor: LYLYK, Pedro, C1428ARJ Buenos Aires (AR); FRID, Noureddine, B-1650 Beersel (BE); GEBHART, Laurence, B-1084 Brussels (BE); KÖNTGES, Nils, B-1180 Brussels (BE)
(74) Representative: Pronovem
(86) International application number: PCT/EP2014/053481
(87) International publication number: WO 2014/128280

(56) References cited:
- WO-A1-99/38998
- WO-A2-2008/017721
- US-A1- 2003 044 408
- US-A1- 2005 049 693
- US-A1- 2008 011 613
- DUNN C J ET AL: "BISPHOSPHONATES: A REVIEW OF THEIR NOVEL ANTI-INFLAMMATORY PROPERTIES AND POTENTIAL FOR THE TREATMENT OF RHEUMATOID ARTHRITIS", IDRUGS, CURRENT DRUGS LTD, GB, vol. 1, no. 4, 1 January 1998 (1998-01-01) , pages 429-441, XP008044461, ISSN: 1369-7056
- KANAME YAMAMOTO ET AL: "Inhibitory effect of bone resorption and inflammation with etidronate therapy in patients with rheumatoid arthritis for 3 years and in vitro assay in arthritis models", RHEUMATOLOGY INTERNATIONAL ; CLINICAL AND EXPERIMENTAL INVESTIGATIONS, SPRINGER, BERLIN, DE, vol. 26, no. 7, 1 May 2006 (2006-05-01), pages 627-632, XP019335375, ISSN: 1437-160X, DOI: 10.1007/S00296-005-0042-Y

## Description

### Technical Field

The present invention relates to a biocompatible coating for an implantable medical device having a metallic part, and more particularly relates to a bisphosphonate-based coating covalently and directly bonded to a surface of the metallic part. This invention also relates a method for producing an implantable device having a metallic part to which a bisphosphonic acid compound is covalently and directly bonded as external coating.

### Background of the invention

Implantable medical devices such as artificial heart valves, vascular prosthesis, stents, orthopedic screws and joint prosthesis often comprise or essentially consist of a metallic substrate to obtain adequate mechanical properties. However, the metallic substrates used for implantable medical devices have disadvantage with regard to their biocompatibility or functionality, in particular, in long-term use.

Implants often trigger inflammatory tissue responses and immune reactions through chemical and/or physical irritation, thus resulting in intolerance reactions in the sense of chronic inflammatory reactions with defensive and rejection reactions, excessive scar tissue production of degradation of tissue. There has therefore been a demand for a coating for the implants that reduces inflammatory responses caused by implantation.

Stents, in general, are endovascular prostheses or implants which are used i.a. for treating stenosis. (Re)stenosis is a narrowing of a blood vessel, leading to restricted blood flow, blockage of the blood and insufficient oxygenation of cardiac tissue, resulting in myocardial ischemia, infarction, cardiac arrhythmia, or cardiac arrest. Basically, stents have a carrier structure suitable for supporting the wall of a vessel in an appropriate manner in order to enlarge the vessel. Restenosis often occurs after a first operation for treating stenosis has been carried out (for example, dilatation of a vessel by a balloon or a classical stent).

U.S. Pat. No. 5741333 discloses a self-expanding bare metal stent, a tiny metal scaffolding that functions to brace the vessel wall, which effectively reduces restenosis and significantly decreases the rates of the major adverse cardiac events, myocardial infarction, and death.

A stated above, stent implantation is often associated with in-stent restenosis, which is defined as late lumen diameter loss greater than 50% within the stent. In-stent restenosis is associated with an excessive proliferation of vascular smooth muscle cells (SMCs), extracellular matrix synthesis and a chronic inflammatory reaction which are believed to be initiated by a deep vascular injury and the endothelial cell denudation created under angioplasty and further by the presence of a foreign metallic device. This complication occurs in about 15 to 30% of the procedures.

Drug-eluting stents (DES), namely, stents combined with drugs, were put on the market to theoretically prevent in-stent restenosis phenomenon through inhibition of SMC proliferation. Generally, there are three components in DES, i.e., a stent platform, drug delivery vehicle such as polymer matrix, and drug such as sirolimus and paclitaxel. Such DESs are supposed to significantly reduce the rate of restenosis compared to bare metal stent. However, an increase in the rate of myocardial infarction and cardiovascular mortality was reported after the implantation of such DES. These events were found to be due to late stent thrombosis, which is often carried out after an acute thrombosis of the artery caused by the platelet aggregation and blood clotting.

In order to alleviate thrombosis and in-stent restenosis, there are various approaches in the state of art for improving the biocompatibility of stents by modifying their surfaces with less thrombotic and inflammatory materials. U.S. Pat. No. 5,891,507, for example, discloses metal stents coated with silicone, polyetrafluoroethylene and biological materials such as heparin or growth factors which increase the biocompatibility of the metal stents.

US2008/0286328A1 and US2005/0049693A1 disclose an implant coated with an amino-bisphosphonate which is not covalently bounded to a surface of the implant. Therefore, the implant releases the bisphosphonate as a pharmaceutical active ingredient) into the immediately surrounding environment of the implant.

US 2008 011613 discloses orthopedic implants which are coated with a bisphosphonate drug such as alendronic acid, to improve bone reconstruction. This document does not disclose any anti-inflamatory or antithrombotic effect.

EP1058343A1 discloses a bisphosphonate coated implant device for inhibiting bone resorption and decreasing inflammatory activity of monocytes and macrophages, thereby improving over time the fixation an integration of the implant device. Two distinguishable bisphosphonate layers are attached to the device through a protein layers immobilized on a surface of the device, releasable therefrom and act as "drug" (active ingredient) locally.

On the other hand, US2003/0044408A1 discloses a metallic frame comprising a composition for delivering a biologically active molecule without the use of polymer and other dense coatings, thereby minimizing an inflammatory response. The surface of the metallic substrate is chemically modified for attachment of proteins, peptides, and pharmaceuticals by using amino-bisphosphonate having a derivatizable functionalities as a linkage compound.

WO2008/017721 discloses a method of coating a metallic substrate with a bisphosphonate by depositing the bisphosphonate on a surface of the substrate in a medium and following by thermal dehydration after removing the medium. It also discloses a metallic substrate coated with the bisphosphonate and the use of the coated substrate in watch- and clock making, the automobile or aeronautical industry, or in electromechanical microsystem or nanosystem to limit sticking forces, to facilitate sliding between two mechanical parts, to limit the spreading of liquid on these service by supporting the epilame effect or to limit the formation of crystallization.

None of prior art discloses that a bisphosphonate as a coating permanently and directly deposited on the metallic substrate of a vascular prosthesis provides the anti-platelet property and the anti-inflammatory property as mechanical property of the coating.

### Summary of the invention

The present invention provides an implantable medical device having a new type of coating on a metallic surface thereof.

The coating according to the present invention comprises a bisphosphonic acid which is covalently and directly bonded to a metallic surface of an implantable medical device and forms a monolayer as external layer of the coating. Some molecules of the bisphosphonic acid may be covalently bonded to another molecule of the bisphosphonic acid in the coating. The coating provides biocompatibility for the metallic surface, resulting in reducing inflammatory responses caused by the implantation. The coating also provides improved platelet-antiadhesion property for the metallic sureface while keeping good compatibility with the platelet poor plasma (PPP) and with the endothelial cells which are required for rapid endothelialization on the coating. As a result, the risk of late thrombosis and in-stent restenosis can be reduced with the coating.

The object of the invention is to put on the market an endoprosthesis that provides increased biocompatibility of its surface, resulting in reducing the risk of a chronic inflammatory reactions, thrombosis, and in-stent restenosis, caused by the implantation.

Another object of the present invention is to provide a coating permanently deposited on a surface of metallic substrate of a vascular endoprosthesis and decreasing platelet-aggregation while keeping rapid promotion of endothelial cells growth thereon, namely anti-platelet and biocompatible coating,
Further another object of the present invention is to provide a coating permanently deposited on a surface of metallic substrate of a vascular endoprosthesis and decreasing cell inflammatory response while keeping rapid promotion of endothelial cells growth thereon, namely anti-inflammatory and biocompatible coating,
The subject of the present invention is defined in the appended independent claims. Preferred embodiments are defined in the dependent claims.

The subject of the present invention is an implantable medical device comprising a metallic substrate and a bisphosphonate. Both phosphorus atom contained in the bisphosphonate are covalently attached to a same carbon atom. The bisphosphonate continuously coats the external surface of the metallic substrate as monolayer and as outermost layer, and at least one phosphonate moiety of a molecule of the bisphosphonate is covalently and directly bonded to the external surface of the metallic substrate and/or covalently bonded to another molecule of the bisphosphonate in the coating.

Another subject of the present invention is an implantable medical device comprising a metallic substrate and a bisphosphonate. The bisphosphonate may consist of the general formula (I). 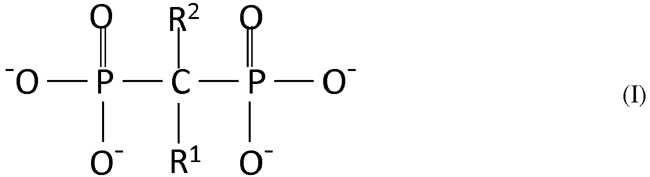
R¹ may represent:
   (i) -C₁₋₅ alkyl, unsubstituted or substituted with -NH₂, pyridyl, pyrrolidyl or-NR³R⁴;
   (ii) -NHR⁵;
   (iii) -SR⁶; or
   (iv) -Cl;
R² may represent -H, -OH, or -Cl;
R³ may represent -H or -C₁₋₅ alkyl;
R⁴ may represent -C₁₋₅ alkyl;
R⁵ may represent -C₁₋₁₀ alkyl or -C₃₋₁₀ cycloalkyl;
R⁶ may represent phenyl.

In another embodiment, the surface phosphorus-atom concentration of the coating of the bisphosphonate substrate is at least 70%P, preferably at least 80 %P.

In another embodiment, the implantable medical device can be selected from the group consisting of vascular endoprosthesis, intraluminal endoprosthesis, stents, coronary stents and peripheral stents.

In another aspect, the present invention provides a method for coating a metallic substrate with a bisphosphonate substrate, comprising the following steps:
(a) providing the metallic substrate;
(b) preparing a liquid carrier comprising a bisphosphonic acid;
(c) immersing the metallic substrate into the liquid carrier; and
(d) covalently immobilizing the bisphosphonic acid compound onto a surface of the metallic substrate in the liquid carrier, and
both phosphorus atom contained in the bisphosphonic acid are covalently attached to same carbon atom.

In one embodiment of the invention, the bisphosphonic acid having the formula (I) or a salt thereof, wherein: 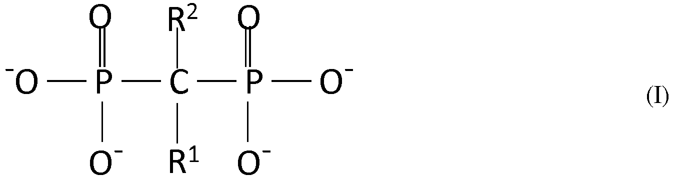
R¹ may represent:
   (i) -C₁₋₅ alkyl, unsubstituted or substituted with -NH₂, pyridyl, pyrrolidyl or-NR³R⁴;
   (ii) -NHR⁵;
   (iii) -SR⁶; or
   (iv) -Cl;
R² may represent -H, -OH, or -Cl;
R³ may represent -H or -C₁₋₅ alkyl;
R⁴ may represent -C₁₋₅ alkyl;
R⁵ may represent -C₁₋₁₀ alkyl or -C₃₋₁₀ cycloalkyl;
R⁶ may represent phenyl.

In another embodiment, the liquid carrier may be heated for at least 24 hours, preferably 48 hours, at a temperature at least 70 °C, preferably at least 80 °C in step (d).

In another embodiment, the metallic substrate in the liquid carrier is subjected to an induction heating in step (d).

Still another embodiment of the invention relates the method, wherein the surface of the metallic substrate to be coated can be subjected to a thermal treatment for promoting the formation of a thick external metal oxide layer thereon, preferably, the thermal treatment comprises a step of heating the metallic substrate to be coated for at least 10 minutes, preferably at least 15 minutes, at a temperature at least 500°C, preferably at least 550°C.

Another subject of the present invention relates to a bisphosphonate for use as anti-inflammatory coating for a metallic surface of an implantable medical device, wherein both phosphorus atoms of the bishosphonate are covalently attached to same carbon atom.

Still another subject of the present invention relates to a bisphosphonate for use as anti-thrombogenic coating for a metallic surface of a endovascular prostheses, wherein both phosphorus atoms of the bishosphonate are covalently attached to same carbon atom.

### Brief description of the Figures

Figure 1 is comparative graph showing platelet rich plasma (PRP) adhesion on a bare-metal surface as comparative example (CEX) and on a bisphosphonate coating according to the invention (INV).
Figure 2 is comparative graph showing platelet poor plasma (PPP) adhesion on a bare-metal surface as comparative example (CEX) and on a bisphosphonate coating according to the invention (INV).
Figure 3 is comparative graph showing relative endothelialisation on a bare-metal surface as comparative example (CEX) and on a bisphosphonate coating according to the invention (INV).
Figure 4 is comparative graph showing relative inflammation with a bare-metal surface as comparative example (CEX) and with a bisphosphonate coating according to the invention (INV).

### Detailed description of the invention

The terms "implantable medical device" and "implant" are used synonymously here and are understood to include medical or therapeutic implants, such as vascular endoprosthesis, intraluminal endoprosthesis, stents, coronary stents, peripheral stents, surgical and/or orthopedic implant for temporary use, such as surgical screws, plates, nails and other fastening means, permanent surgical or orthopedic implants, such as bone prosthesis or joint prosthesis.

The term of "vascular endoprosthesis" are used synonymously here and are understood to include stents, stentgraft, filter, heart valve, coronary stents and peripheral stents.

The implantable medical device according to the present invention comprises, or essentially consists of, a metallic substrate which is selected from the group consisting of iron, magnesium, nickel, tungsten, titanium, zirconium, niobium, tantalum, zinc or silicon and, if necessary, a second component of one or several metals from the group consisting of lithium, sodium, potassium, calcium, manganese, iron or tungsten, preferably of a zinc-calcium alloy. In a further practical example, the metallic substrate consists of a memory effect material of one or several materials from the group consisting of nickel titanium alloys and copper zinc aluminium alloys, but preferably of nitinol. In a further practical example, the metallic substrate of the medical device consists of stainless steel, preferably of a Cr-Ni-Fe steel, in this case, preferably the alloy 316L, or a Co-Cr steel such as Phynox. In preferred embodiments of the present invention, the implantable medical devices are stents, in particular metal stents, preferably self-expanding stents.

This invention is related to a coating for a metallic substrate comprising a bisphosphonate layer derived from a bisphosphonic acid, the two phosphorus atoms of the bisphosphonic acid are covalently attached to same carbon atom.

U.S. Pat. No. 5,431,920 discloses a composition in dosage form comprising a bisphosphonic acid as active ingredient for treating metabolic bone diseases such as osteoporosis.

European Pat No. 1,508,343 discloses a multilayer coating for an implant device for use of bone fixation. The coating comprises two types of bisphosphate layers; one is designed to release from the multilayer coating immediately after the implant device has been inserted and the other designed slowly released over time for the long-time use of the implant device. The latter type of bisphosphate layer is strongly bonded to a plurality of protein layers which are immobilized on a surface of the implant device. The former type of bisphosphate is loosely bonded to the latter type of layers as outermost layer of the coating.

According to the present invention, a bisphosphonate coats a metallic surface of an implantable medical device as monolayer and as outermost layer. At least one phosphonate moiety of the bisphosphonate is covalently and directly bonded to the metallic surface. Some molecules of the bisphosphonate may covalently bond to another molecule of the bisphosphonate in the coating. Therefore, after implantation, the bisphosphonate or bisphosphonic acid is not released by the coating as an active ingredient but permanently stays on the surface as a part of the coating. Surprisingly, the mere presence of the bisphosphonate layer provides biocompatibility to the coating and reduces the inflammatory response and/or promotes the formation of an endothelial cell layer on the coating while significantly decreasing the activation and adhesion of platelet. As a result, the risks of restenosis and thrombosis are strongly reduced.

"Phosphate group" means a functional group comprising phosphorus attached to four oxygens and with net negative charge, thus presented as PO₄⁻. "Phosphonic acid" is an organic compound containing C-PO(OH)₂ group. "Phosphonate" is a salt or ester of an phosphonic acid and has a general formula R¹-PO(OR²)₂ group (where R¹ represents alkyl or aryl and R² represents alkyl, aryl or a counter cation of the salts). For purposes of the present invention, "bisphosphonate" refers to compounds having two phosphonate (PO₃) groups in the molecule. Bisphosphonates according to the present invention have a common P-C-P "backbone", namely, the two phosphonate (PO₃) groups covalently linked to one carbon atom.

In preferred embodiments of the present invention, a bisphosphonic acid used for providing the bisphosphonate monolayer on the metallic surface of the implantable medical device has the general formula (I) or a salt thereof. 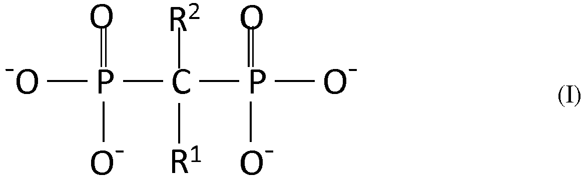 In the formula (I) R¹ represents -C₁₋₅ alkyl (which is unsubstituted or substituted with -NH₂, pyridyl, pyrrolidyl or -NR³R⁴), -NHR⁵, -SR⁶ or -Cl; R² represents -H, -OH, or -Cl; R³ represents -H or -C₁₋₅ alkyl; R⁴ represents -C₁₋₅ alkyl; R⁵ represents -C₁₋₁₀ alkyl or -C₃₋₁₀ cycloalkyl; R⁶ represents phenyl.

As used herein, "alkyl" is intended to include both branched- and straight-chain saturated aliphatic hydro carbon groups having the specified number of carbon atoms; "cycloalkyl" is intended to include saturated ring groups, such as cyclopropyl, cyclobutyl, cyclopentyl, cyclohexyl and cycloheptyl.

In preferred embodiments of the present invention, the bisphosphonic acid can be selected from the group consisting of 1-hydroxyethylidene-1,1-diphosphonic acid (etidronic acid), alendronic acid, clodronic acid, pamidronic acid, tiludronic acid and risedronic acid, preferably 1-hydroxyethylidene-1,1-diphosphonic acid (etidronic acid).

The bisphosphonate in the coating according to the present invention continuously coats the external surface of the metallic substrate as monolayer and as outermost layer, and at least one phosphonate moiety of each molecule of the bisphosphonate is covalently and directly bonded to the external surface of the metallic substrate and/or covalently bonded to another molecule of the bisphosphonate in the coating.

Induction heating is a widely used method to heat metallic substrates directly and contactless by electromagnetic induction, where eddy currents are generated within the metal and resistance leads to Joule heating of the metal. An induction heater (for any process) consists of an electromagnet such as coil, through which a high-frequency alternating current (AC) is passed.

In a preferred embodiment, a container made of non-conductive material comprising a metallic substrate to be coated and a liquid carrier containing the bisphosphonic acid compound is located inside of a conductive coil. The metallic substrate is subject to the eddy current produced by passing through the coil a AC with a power output of between 50 and 500 kW and a frequency between 150 and 250 kHz, so as to obtain a bisphosphate layer on a surface of the metallic substrate with a desirable thickness. The concentration of the bisphosphonic acid compound is between 10⁻² and 10⁻⁴ mol/l, preferably 10⁻³ mol/l. The bisphosphonic acid compound is preferably dissolved in the liquid carrier. The liquid carrier may be alcohol such as methanol and ethanol and isopropanol, THF, DMF, water or a mixture thereof.

In a further preferred embodiment, a metallic substrate to be coated is immersed in a liquid carrier containing the bisphosphonic acid at a concentration between 10⁻² and 10⁻⁴ M and the liquid carrier is heated by conventional means for at least 24 h at a temperature of at least 70 °C.

The metallic substrate may be subject to a thermal treatment (TT) so as to promote the formation of a thick oxidized layer on the metallic substrate.

Surface compositions (total atomic concentration %) of bisphosphate monolayers on a metallic substrate has been measured with X-ray photoelectron spectroscopy (XPS). The surface phosphorus-atom concentration of the coating of the bisphosphate substrate is at least 70 %P, preferably at least 90 %P.

### Examples

### Example 1: (formation of a coating)

5 mm Phynox stents in 2 cm long were provided as medical device to be coated. The Phynox surface was modified by a thermal treatment (TT) in an oven for 15 min at 550 °C, and then a chemical treatment (CT, immersing the stent into a solution of 0.1% HF and 20% HNO₃ for 15 min and then immersing the stent in solution of 2.5% HNO₃ for 30 min) was performed. The stents were subjected to an ethylene oxide sterilization process before surface treatment. Etidronic acid (EA) aqueous solution was obtained by dilution 1000x of the mother solution (60%v/v). The stent was immersed into the solution and heated at 80 °C for 48 hours.

### Example 2: (induction heating)

5 mm Phynox stents in 2 cm long stent were provided as medical device to be coated. The Phynox surface was modified by a thermal treatment (TT) in an oven for 15 min at 550 °C, and then a chemical treatment (CT, immersing the stent into a solution of 0.1% HF and 20% HNO3 for 15 min and then immersing the stent in solution of 2.5% HNO₃ for 30 min) was performed. Etidronic acid aqueous solution was prepared at a concentration of 10⁻³ M. The stents were immersed into the solution and subjected to the induction heating with Ambrell EasyHeat induction heating system with a power output of 110 kW and a frequency of 198 kHz. The used solenoid was composed of 7 spires with an internal diameter of 9 cm.

### Example 3: (Platelet adhesion)

Blood was collected in 3.6ml sodium citrate 3.5% vials to prevent coagulation. Full blood was being centrifuged at two different rotation speeds in order to obtain two different types of plasma. The first type (a plasma rich in platelets (PRP)) was obtained by centrifugation at 900 rpm, and the second type (a plasma poor in platelets (PPP)) was obtained by centrifugation at 1500 rpm. In order to have sufficient volume for the test, plasma was diluted in HBSS (Hank's buffered salt solution). Platelets adhesion was assessed by spectrophotometry. The amount of total protein was evaluated by measurement of the absorbance at 280 nm. The bare-metal stents and the ones with the EA coating were prepared as described in example 1 and immersed in one of the two plasma solutions for 24 hours, at 37°C and under constant agitation (STAT-FAX 2200 Thermostated agitator). When completed the stents are rinsed with HBSS buffer and then treated with 125 µl lysis solution. Total absorbance at 280 nm is than being measured. Raw data were normalized by dividing every sample value by the average of the control for every experiment (PRP and PPP).

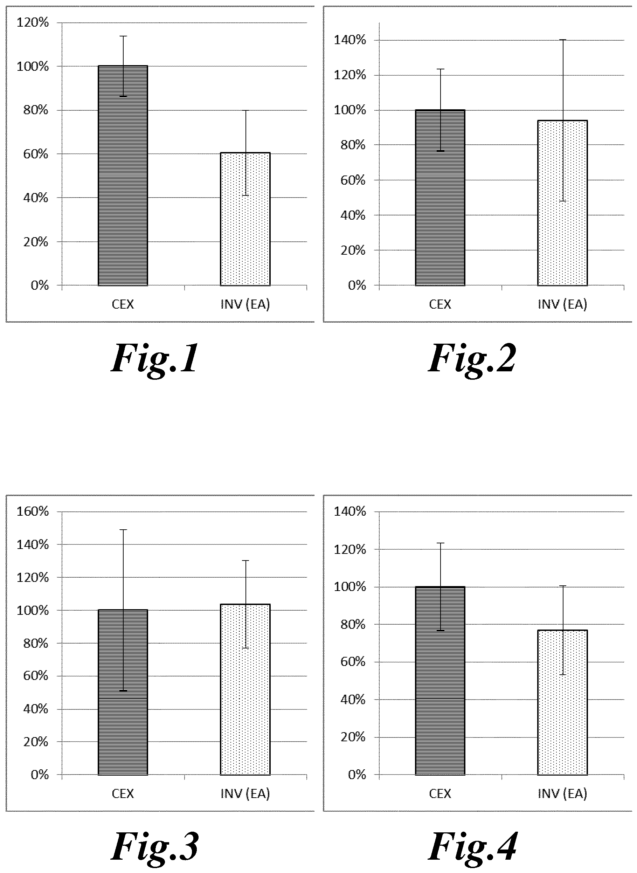

Figs. 1 and 2 show respectively PRP and PPP adhesions to the bare-metal surface (CEX) and to the EA coating according to the invention (INV). The difference between the PRP and the PPP value reflects the amount of platelets adsorbed on the surface.

Conclusion: An important (40% !) decrease of platelet aggregation was observed on the EA coating according to the invention compared to the bare-metal surface.

### Example 4: (Endothelial cells growth: endothelialisation)

The bare-metal stents and the ones with the EA coating were prepared as described in example 1 and incubated with 70.000 cells (EA.hY926) per well for 7 days (37°C, CO₂ 5%). This large amount of cells was meant to enhance the probability for cells to be trapped into the meshing of the stents. 80% (4ml out of 5ml) of the culture medium was replaced twice during the incubation period (after 1 and 4 days). When completed the stents are rinsed with HBSS buffer and then treated with 125 µl lysis solution. Total absorbance at 280 nm is than being measured.

Figure 3 shows relative endothelialisation on the bare-metal surface (CEX) and on the EA coating according to the invention (INV).

Conclusion: a same level of endothelial cells growth was observed on the EA coating as compared to the bare-metal surface. Thus, desirable ability of wall cell regeneration is not reduced while decreasing platelet aggregation.

### Example 5: (Inflammation induction)

The IL-8 values obtained with the bare-metal stents and with the EA coating stents prepared as described in example 1 were measured in the medium using a regular ELISA set in accordance with the instructions provided.

Figure 4 shows relative inflammation with the bare-metal surface (CEX) and with the EA coating according to the invention (INV).

Conclusion: The inflammatory response induced with the EA coating was significantly improved, i.e., 23% lower, compared to the bare-metal surface.

Accordingly, the bisphosphonate coating according to the present invention exhibited a significant decease of platelet adhesion and cell inflammatory response compared to the bare-metal surface while keeping rapid promotion of endothelial cell growth thereon.

## Claims

1. A vascular endoprosthesis comprising:
(a) a metallic substrate; and
(b) a bisphosphonate having the general formula (I), 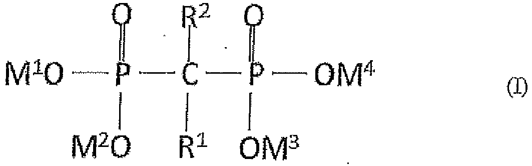 **characterized in that**:
R¹ represents (i) -C₁₋₅ unsubstituted alkyl, or (ii) -Cl;
R² represents -H, -OH, or -Cl;
M¹, M², M³, M⁴ represent independently of one another a hydrogen atom or any metallic atom,
the bisphosphonate continuously coating the external surface of the metallic substrate as monolayer and as outermost layer, and at least one phosphonate moiety of the bisphosphonate being covalently and directly bonded to the external surface of the metallic substrate in the coating.

2. The vascular endoprosthesis according to claim 1, wherein R¹ represents -CH₃ and R² represents -OH.

3. The vascular endoprosthesis according to claim 1 or 2, wherein the surface phosphorus-atom concentration of the coating of the bisphosphonate is at least 70%P, preferably at least 80 %P.

4. The vascular endoprosthesis according to any one of preceding claims, selected from the group consisting of stents, stentgraft, filter, heart valve, coronary stents and peripheral stents.

5. A composition comprising bisphosphonate having the general formula (I) for use as anti-inflammatory coating for a metallic surface of a vascular endoprosthesis, 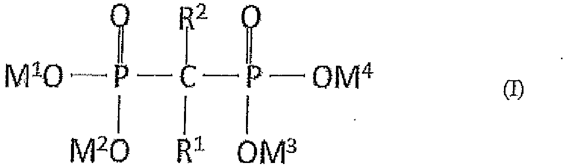 wherein:
R¹ represents (i) -C₁₋₅ unsubstituted alkyl, or (ii)-Cl;
R² represents -H, -OH, or -Cl;
M¹, M², M³, M⁴ represent independently of one another a hydrogen atom or any metallic atom,
the bisphosphonate continuously coating the external surface of the metallic substrate as monolayer and as outermost layer, and at least one phosphonate moiety of the bisphosphonate being covalently and directly bonded to the external surface of the metallic substrate in the coating.

6. The composition for use according to claim 5, wherein R¹ represents -CH₃ and R² represents -OH.

7. A composition comprising bisphosphonate having the general formula (I) for use as anti-thrombogenic coating for a metallic surface of a vascular endoprosthesis, 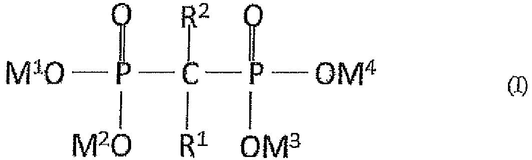 wherein:
R¹ represents (i) -C₁₋₅ unsubstituted alkyl, or (ii)-Cl;
R² represents -H, -OH, or -Cl;
M¹, M², M³, M⁴ represent independently of one another a hydrogen atom or any metallic atom,
the bisphosphonate continuously coating the external surface of the metallic substrate as monolayer and as outermost layer, and at least one phosphonate moiety of the bisphosphonate being covalently and directly bonded to the external surface of the metallic substrate in the coating.

8. The composition for use according to claim 7, wherein R¹ represents -CH₃ and R² represents -OH.

9. Use of bisphosphonate having the general formula (I) for the preparation of an anti-inflammatory
coating for a metallic surface of a vascular endoprosthesis, 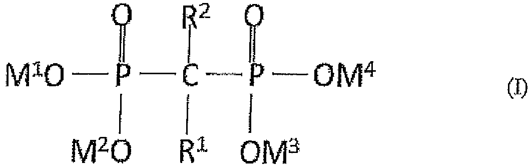 wherein:
R¹ represents (i) -C₁₋₅ unsubstituted alkyl, or (ii) -Cl;, preferably -CH₃;
R² represents -H, -OH, or -Cl, preferably -OH;
M¹, M², M³, M⁴ represent independently of one another a hydrogen atom or any metallic atom,
the bisphosphonate continuously coating the external surface of the metallic substrate as monolayer and as outermost layer, and at least one phosphonate moiety of the bisphosphonate being covalently and directly bonded to the external surface of the metallic substrate in the coating.

10. Use of bisphosphonate having the general formula (I) for the preparation of an anti-platelet coating for
a metallic surface of a vascular endoprosthesis, 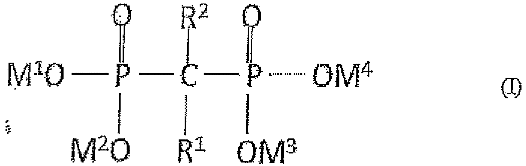 wherein:
R¹ represents (i) -C₁₋₅ unsubstituted alkyl, or (ii)-Cl;, preferably -CH₃;
R² represents -H, -OH, or -Cl, preferably -OH;
M¹, M³, M³, M⁴ represent independently of one another a hydrogen atom or any metallic atom,
the bisphosphonate continuously coating the external surface of the metallic substrate as monolayer and as outermost layer, and at least one phosphonate moiety of the bisphosphonate being covalently and directly bonded to the external surface of the metallic substrate in the coating.

## Patentansprüche

1. Gefäßendoprothese, die umfasst:
(a) ein metallisches Substrat; und
(b) ein Bisphosphonat mit der allgemeinen Formel (I), 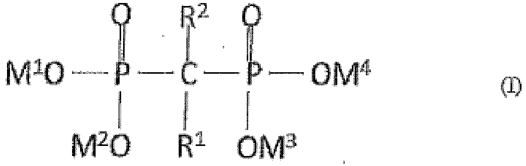 **dadurch gekennzeichnet, dass**:
R¹ (i) unsubstituiertes -C₁₋₅-Alkyl, oder (ii) -Cl darstellt;
R² -H, -OH oder -Cl darstellt;
M¹, M², M³, M⁴ unabhängig voneinander ein Wasserstoffatom oder ein beliebiges metallisches Atom darstellen,
wobei das Bisphosphonat die Außenfläche des metallischen Substrats als Monoschicht und als äußerste Schicht durchgehend bedeckt und wobei zumindest eine Phosphonateinheit des Bisphosphonats kovalent und direkt an die Außenfläche des metallischen Substrats in der Beschichtung gebunden ist.

2. Gefäßendoprothese nach Anspruch 1, wobei R¹ -CH₃ darstellt und R² -OH darstellt.

3. Gefäßendoprothese nach Anspruch 1 oder 2, wobei die Oberflächenphosphoratomkonzentration der Beschichtung des Bisphosphonats zumindest 70 % P, vorzugsweise zumindest 80 % P, beträgt.

4. Gefäßendoprothese nach einem der vorstehenden Ansprüche, die aus der Gruppe ausgewählt ist, bestehend aus Stents, Stenttransplantaten, Filter, Herzklappe, Koronarstents und peripheren Stents.

5. Zusammensetzung, die Bisphosphonat mit der allgemeinen Formel (I) umfasst, zur Verwendung als entzündungshemmende Beschichtung für eine metallische Oberfläche einer Gefäßendoprothese, 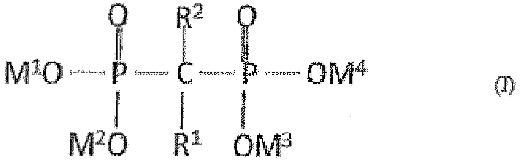 wobei:
R¹ (i) unsubstituiertes -C₁₋₅-Alkyl, oder (ii) -Cl darstellt;
R² -H, -OH oder -Cl darstellt;
M¹, M², M³, M⁴ unabhängig voneinander ein Wasserstoffatom oder ein beliebiges metallisches Atom darstellen,
wobei das Bisphosphonat die Außenfläche des metallischen Substrats als Monoschicht und als äußerste Schicht durchgehend bedeckt und wobei zumindest eine Phosphonateinheit des Bisphosphonats kovalent und direkt an die Außenfläche des metallischen Substrats in der Beschichtung gebunden ist.

6. Zusammensetzung zur Verwendung nach Anspruch 5, wobei R¹ -CH₃ darstellt und R² -OH darstellt.

7. Zusammensetzung, die Bisphosphonat mit der allgemeinen Formel (I) umfasst, zur Verwendung als antithrombogene Beschichtung für eine metallische Oberfläche einer Gefäßendoprothese, 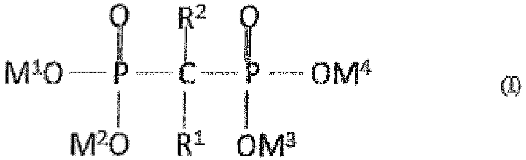 wobei:
R¹ (i) unsubstituiertes -C₁₋₅-Alkyl; oder (ii) -Cl darstellt;
R² -H, -OH oder -Cl darstellt;
M¹, M², M³, M⁴ unabhängig voneinander ein Wasserstoffatom oder ein beliebiges metallisches Atom darstellen,
wobei das Bisphosphonat die Außenfläche des metallischen Substrats als Monoschicht und als äußerste Schicht durchgehend bedeckt und wobei zumindest eine Phosphonateinheit des Bisphosphonats kovalent und direkt an die Außenfläche des metallischen Substrats in der Beschichtung gebunden ist.

8. Zusammensetzung zur Verwendung nach Anspruch 7, wobei R¹ -CH₃ darstellt und R² -OH darstellt.

9. Verwendung von Bisphosphonat mit der allgemeinen Formel (I) zur Herstellung einer entzündungshemmenden Beschichtung für eine metallische Oberfläche einer Gefäßendoprothese, 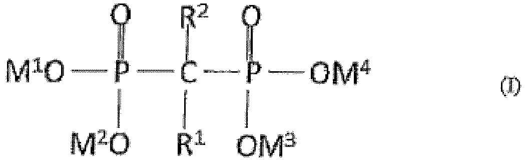 wobei:
R¹ (i) unsubstituiertes -C₁₋₅-Alkyl; oder (ii) -Cl; vorzugsweise -CH₃, darstellt;
R² -H, -OH oder -Cl, vorzugsweise -OH, darstellt;
M¹, M², M³, M⁴ unabhängig voneinander ein Wasserstoffatom oder ein beliebiges metallisches Atom darstellen,
wobei das Bisphosphonat die Außenfläche des metallischen Substrats als Monoschicht und als äußerste Schicht durchgehend bedeckt und wobei zumindest eine Phosphonateinheit des Bisphosphonats kovalent und direkt an die Außenfläche des metallischen Substrats in der Beschichtung gebunden ist.

10. Verwendung von Bisphosphonat mit der allgemeinen Formel (I) zur Herstellung einer Anti-Blutplättchen-Beschichtung für eine metallische Oberfläche einer Gefäßendoprothese, 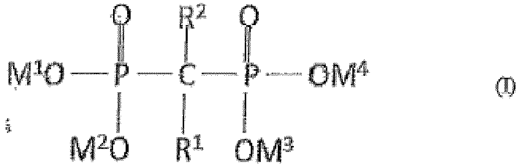 wobei:
R¹ (i) unsubstituiertes -C₁₋₅-Alkyl; oder (ii) -Cl); vorzugsweise -CH₃, darstellt;
R² -H, -OH oder -Cl, vorzugsweise -OH, darstellt;
M¹, M², M³, M⁴ unabhängig voneinander ein Wasserstoffatom oder ein beliebiges metallisches Atom darstellen,
wobei das Bisphosphonat die Außenfläche des metallischen Substrats als Monoschicht und als äußerste Schicht durchgehend bedeckt und wobei zumindest eine Phosphonateinheit des Bisphosphonats kovalent und direkt an die Außenfläche des metallischen Substrats in der Beschichtung gebunden ist.

## Revendications

1. Endoprothèse vasculaire comprenant :
(a) un substrat métallique ; et
(b) un bisphosphonate ayant la formule générale (I), 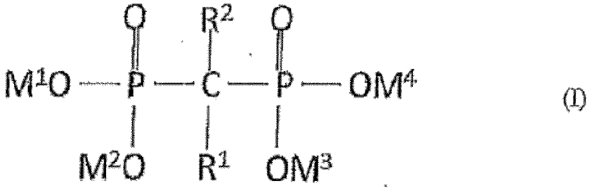 **caractérisée en ce que** :
R¹ représente (i) -alkyle non substitué en C₁₋₅ ou (ii) -Cl ;
R² représente -H, -OH ou -Cl ;
M¹, M², M³, M⁴ représentent indépendamment les uns des autres un atome d'hydrogène ou un quelconque atome métallique,
le bisphosphonate recouvrant de manière continue la surface externe du substrat métallique sous forme de monocouche et sous forme de couche la plus externe, et au moins une fraction phosphonate du bisphosphonate étant liée de manière covalente et de manière directe à la surface externe du substrat métallique dans le revêtement.

2. Endoprothèse vasculaire selon la revendication 1, dans laquelle R¹ représente -CH₃ et R² représente -OH.

3. Endoprothèse vasculaire selon la revendication 1 ou 2, dans laquelle la concentration en atomes de phosphore en surface du revêtement du bisphosphonate est d'au moins 70 % de P, de préférence d'au moins 80 % de P.

4. Endoprothèse vasculaire selon l'une quelconque des revendications précédentes, choisie dans le groupe constitué par les stents, les stent-greffons, un filtre, une valvule cardiaque, les stents coronaires et les stents périphériques.

5. Composition comprenant un bisphosphonate ayant la formule générale (I) pour une utilisation en tant que revêtement anti-inflammatoire pour une surface métallique d'une endoprothèse vasculaire, 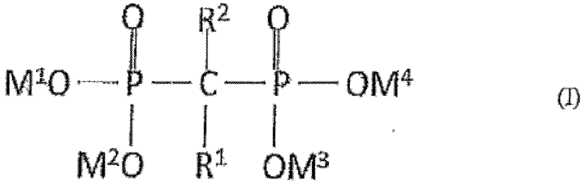 dans laquelle :
R¹ représente (i) -alkyle non substitué en C₁₋₅ ou (ii) -Cl ;
R² représente -H, -OH ou -Cl ;
M¹, M², M³, M⁴ représentent indépendamment les uns des autres un atome d'hydrogène ou un quelconque atome métallique,
le bisphosphonate recouvrant de manière continue la surface externe du substrat métallique sous forme de monocouche et sous forme de couche la plus externe, et au moins une fraction phosphonate du bisphosphonate étant liée de manière covalente et de manière directe à la surface externe du substrat métallique dans le revêtement.

6. Composition pour une utilisation selon la revendication 5, dans laquelle R¹ représente -CH₃ et R² représente -OH.

7. Composition comprenant du bisphosphonate ayant la formule générale (I) pour une utilisation en tant que revêtement anti-thrombogène pour une surface métallique d'une endoprothèse vasculaire, 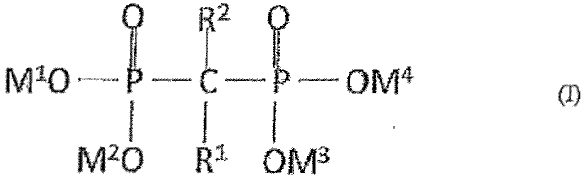 dans laquelle :
R¹ représente (i) -alkyle non substitué en C₁₋₅ ou (ii) -Cl ;
R² représente -H, -OH ou -Cl ;
M¹, M², M³, M⁴ représentent indépendamment les uns des autres un atome d'hydrogène ou un quelconque atome métallique,
le bisphosphonate recouvrant de manière continue la surface externe du substrat métallique sous forme de monocouche et sous forme de couche la plus externe, et au moins une fraction phosphonate du bisphosphonate étant liée de manière covalente et de manière directe à la surface externe du substrat métallique dans le revêtement.

8. Composition pour une utilisation selon la revendication 7, dans laquelle R¹ représente -CH₃ et R² représente -OH.

9. Utilisation de bisphosphonate ayant la formule générale (I) pour la préparation d'un revêtement anti-inflammatoire pour une surface métallique d'une endoprothèse vasculaire, 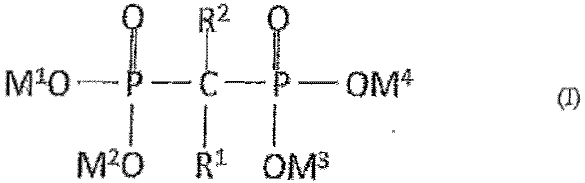 dans laquelle :
R¹ représente (i) -alkyle non substitué en C₁₋₅ ou (ii) -Cl ; de préférence -CH₃ ;
R² représente -H, -OH ou -Cl ; de préférence -OH ;
M¹, M², M³, M⁴ représentent indépendamment les uns des autres un atome d'hydrogène ou un quelconque atome métallique,
le bisphosphonate recouvrant de manière continue la surface externe du substrat métallique sous forme de monocouche et sous forme de couche la plus externe, et au moins une fraction phosphonate du bisphosphonate étant liée de manière covalente et de manière directe à la surface externe du substrat métallique dans le revêtement.

10. Utilisation de bisphosphonate ayant la formule générale (I) pour la préparation d'un revêtement anti-plaquettaire pour une surface métallique d'une endoprothèse vasculaire, 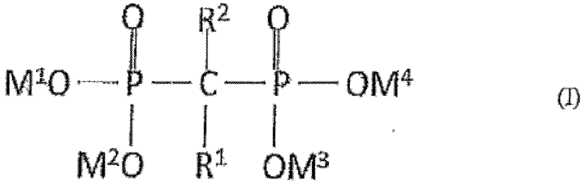 dans laquelle :
R¹ représente (i) -alkyle non substitué en C₁₋₅ ou (ii) -Cl ; de préférence -CH₃ ;
R² représente -H, -OH ou -Cl ; de préférence -OH ;
M¹, M², M³, M⁴ représentent indépendamment les uns des autres un atome d'hydrogène ou un quelconque atome métallique,
le bisphosphonate recouvrant de manière continue la surface externe du substrat métallique sous forme de monocouche et sous forme de couche la plus externe, et au moins une fraction phosphonate du bisphosphonate étant liée de manière covalente et de manière directe à la surface externe du substrat métallique dans le revêtement.
